# EUROPEAN PATENT APPLICATION

(11) **EP 4 563 563 A1**
(43) Date of publication of application: **04.06.2025**
(21) Application number: 23845758.4
(22) Date of filing: 17.07.2023
(51) Int. Cl.: C07C 11/04, C07C 1/24, C12P 7/10

(54) **PRODUCTION OF BIOETHYLENE BY CATALYTICALLY DEHYDRATING ADVANCED BIOETHANOL FROM OFMSW**

(30) Priority: 28.07.2022 ES 202230695
(71) Applicant: Consejo Superior De Investigaciones Científicas - CSIC, 28006 Madrid (ES); Perseo Biotechnology S.L., 46250 L'Alcúdia - Valencia (ES)
(72) Inventor: PEINADO CEBRIÁN, Cristina, 28049 Madrid (ES); ROJAS MUÑOZ, Sergio, 28049 Madrid (ES); CAMPOS MARTÍN, Jose Miguel, 28049 Madrid (ES); COLL LOZANO, Caterina, 46250 L'Alcúdia - Valencia (ES); LATORRE SÁNCHEZ, Marcos, 46250 L'Alcúdia - Valencia (ES); DURÁ ÁLVAREZ, Alejandro, 46250 L'Alcúdia - Valencia (ES)
(74) Representative: Pons
(86) International application number: PCT/ES2023/070459
(87) International publication number: WO 2024/023384

(57) **Abstract**

The present invention relates to a recovery process for obtaining bioethylene by catalytically dehydrating advanced bioethanol from the organic fraction of municipal solid waste (OFMSW), which will be referred to as such in this patent, said process being an example of a circular economy.

## Description

The present invention relates to a recovery process for obtaining bioethylene by catalytically dehydrating advanced bioethanol from the organic fraction of municipal solid waste (OFMSW), which will be referred to as such in this patent, said process being an example of a circular economy.

### BACKGROUND OF THE INVENTION

Due to rapid population growth, municipal solid waste (MSW) has contributed significantly to the total amount of waste generated by our society. Today, in Europe, every inhabitant generates 0.5 tonnes of MSW per year on average, increasing at an annual rate of 10%. Around 40-50% of this is organic waste. This organic fraction mainly contains carbohydrates, proteins and lipids, which are useful feedstocks that can be converted into valuable products. Their recovery will help to address environmental pollution, but also contributes to the transition from a linear economy to a renewable circular economy. Digestion and composting have helped reduce the biodegradable fraction of MSW sent to landfill. The low economic value of compost and biogas limits the sustainable implementation of selective supply systems, as the increase in environmental taxes on citizens (waste) is necessary to meet significant logistics costs.

Ethylene is the most consumed organic feedstock in the chemical industry. Ethylene production worldwide was 201 million metric tonnes in 2020, and more than 30% of the petrochemical industry is derived from ethylene. Ethylene production today is based on feedstocks derived from oil or natural gas; therefore, a methodology that allows ethylene to be obtained in a renewable manner is of great interest to the industry.

Studies by Chung-Yen Wu et al. [Ethylene Formation from Ethanol Dehydration Using ZSM-5 Catalyst, ACS Omega (2017) 2, 8, 4287-4296] describing a process for obtaining ethylene from ethanol, characterised in that it comprises a step of putting evaporated ethanol in contact with a solid acid catalyst, selected from ZSM-5 zeolite, modified ZSM-5 zeolite and aluminium oxide, at a temperature between 220 and 280°C and 1 atm pressure, and studies by Jiandong Bi et al. [High effective dehydration of bio-ethanol into ethylene over nanoscale HZSM-5 zeolite catalysts, Catalysis Today, Volume 149, Issues 1-2, 2010, Pages 143-147] describing the production of ethylene from bioethanol in the gaseous state against a solid acid catalyst, HZSM-5, at a temperature below 300°C, preferably at 240°C, with a conversion of more than 99%, both have a problem in that they use a diluent in the ethanol to ethylene dehydration reaction, in some cases nitrogen and in other cases water. This makes the process and therefore the cost of ethylene more expensive because additional separation operations and larger reactor sizes are required for the same ethanol flow rate and space velocity. And therefore, there are higher operating costs.

Moreover, the study by Abas Mohsenzadeh et al. [Bioethylene Production from Ethanol: A Review and Techno-economical Evaluation, ChemBioEng Rev 2017, 4, No. 2, 75-91] describes processes for obtaining ethylene from ethanol, but in the processes described and analysed, co-products are produced that hinder ethylene purification. In this case, carbon oxides (CO₂ and CO) are particularly important, and for that reason, the process requires a step of caustic washing of ethylene.

To date, no comprehensive process has been described for obtaining bioethylene from the organic fraction of municipal solid waste without the use of carrier gases or eluents or energy-intensive purification steps after obtaining the bioethylene.

### DESCRIPTION OF THE INVENTION

In a first aspect, the present invention relates to a process for obtaining bioethylene from an organic fraction of municipal solid waste, characterised in that it comprises the following steps:
a) pretreating the organic fraction mechanically of municipal solid waste to reduce its particle size to between 1 cm and 6 cm, preferably between 1 cm and 4 cm;
b) mixing with an inorganic mineral acid in a concentration of between 0.5% and 3% by volume of the organic fraction of municipal solid waste obtained in step (a) and heating for 30 to 90 minutes at a temperature of between 100°C and 200°C;
c) mixing the product obtained in (b) with water in a percentage of solids of between 8% and 35% and subsequently grinding to a particle size of less than 10 mm, heating between 40°C and 70°C and adjusting the pH between 4 and 6
d) adding an enzyme cocktail comprising at least one cellulase and one amylase to the acid solution obtained in (c) between 0.1% and 2% by weight of said acid solution obtained in (c), and let it react for 3 to 24 hours, subsequently reducing the temperature to between 30 and 40°C, adding yeasts (0.1-1 g/l) and carrying out simultaneous fermentation and saccharification for 24 to 48 hours, wherein floating materials and heavy materials are additionally separated during fermentation and saccharification;
e) separating the broth obtained in (d) by means of a solid/liquid method
f) reducing the water content of the liquid obtained in (e) by distillation; and
g) putting the bioethanol obtained in (f) in gaseous form in the absence of any additional eluent or carrier gas in contact with a solid acid catalyst at a temperature of between 190°C and 250°C and a pressure of between 1 and 10 bar, and separating the dehydration compounds obtained by means of the liquid/gas separation method.

The advantages of this process are that the distillate of step (f) yields up to a bioethanol concentration of more than 95% by weight of bioethanol with respect to the water it contains. Furthermore, no use of diluents or carriers is required, but rather there is direct use of the bioethanol produced (step (e)) and reduced in water content by distillation (step (f)) azeotropically for step (g), in addition to the absence of generating co-products obtained in step (g) so that the process of purifying the bioethylene obtained is simplified and is much simpler in number of steps and expenditure of energy. Therefore, this invention makes it possible to obtain polymerisation grade bioethylene directly. Therefore, the reaction products do not include CO or CO₂. The catalyst activity is also very stable, being suitable for months of continuous work, as well as in plant shutdown and restart conditions.

In the present invention, "organic fraction of municipal solid waste or municipal biowaste" means biodegradable garden and park waste, food and kitchen waste from households, offices, restaurants, wholesale, canteens, caterers and retail premises, and comparable waste from food processing plants, as set out in EU Directive 851/2018.

In the present invention, "enzyme cocktail" means a mixture comprising at least one enzyme for cellulose hydrolysis and one enzyme for starch hydrolysis. Cellulases act to degrade cellulose, involving, among others but not limited to the following: cellobiohydrolases (CBH), endoglucanases (EG), type 1 and 2 lytic monooxygenases (LPMO1 and LPMO2), wherein EGs and PMOs internally cleave the cellulose chains releasing chain ends that are targeted by CBHs. CBHs generate cellobiose or oxidised cellobiose that are subsequently hydrolysed by β-glucosidase, and, furthermore, also comprise cellobiose dehydrogenase (CDH) which is a potential electron donor for LPMOs; and wherein amylases include, but are not restricted to: α-amylases (EC 3.2.1.1, also called endoamylase), β-amylases (EC 3.2.1.2), amyloglucosidases (EC 3. 2.1.3), α-glucosidases (EC 3.2.1.20), pullulanases (EC 3.2.1.41), maltotetraohydrolases (EC 3.2.1.60) and isoamylases (EC 3.2.1.68). α-amylases catalyse the hydrolysis of internal α-1,4-glycosidic bonds in low molecular weight products, such as glucose, maltose and short oligomers up to maltohexose units. β-amylases are exohydrolase enzymes that act from the non-reducing end of a polysaccharide chain by hydrolysing α-1,4-glycosidic bonds to yield successive maltose units. Amyloglucosidases attack α-1,4-glycosidic bonds from the non-reducing end, releasing β-D-glucose. α-glucosidases cleave both α-1,4- and α-1,6-glycosidic bonds on the outer glucose residues of amylose or amylopectin from the non-reducing end of the starch molecule. Pullulanases (also known as α-dextrin-6-glucanohydrolases or amylopectin-6-glucanohydrolases) catalyse the hydrolysis of the α-1,6-bonds in pullulan, a linear α-glucan polymer consisting essentially of maltotriosyl units connected by α-1,6-glycosidic bonds. Maltotetrahydrolases hydrolyse the α-1,4-glycosidic bonds to remove successive maltotetraose residues from the non-reducing chain ends. Isoamylases, similar to pullulanases, are able to hydrolyse the α-1,6-glucosidic bonds in amylopectins and are the only enzymes known to completely debranch glycogen. In addition to all the enzymes mentioned above, lytic polysaccharide monooxygenases (LPMOs) have been recently reported to contribute to starch hydrolysis.

In the present invention, "pre-hydrolysis" means an enzymatic hydrolysis step prior to the addition of the yeasts at a temperature between 40 and 70°C.

In the present invention, "simultaneous fermentation and saccharification" means the process in which the enzymes release fermentable sugars (saccharification) and said sugars are consumed by yeasts as they are generated and transformed to ethanol at a temperature between 30 and 40°C.

In the present invention, "additional eluent or carrier gas" means any stream of water or inert gas (nitrogen, argon, helium, etc.) in gas form that is added to the bioethanol stream to decrease the bioethanol concentration and increase the total flow through the reactor.

In a preferred embodiment of the process, the inorganic mineral acid of step (b) is selected from sulphuric acid, nitric acid, hydrochloric acid, phosphoric acid and any combination thereof.

In another preferred embodiment of the process, the solid/liquid separation of step (e) is carried out by a mechanical pressure method based on a rotating trommel screen and screw press.

In a preferred embodiment, the distillation of step (f) is carried out by a 2- to 4-column system in a temperature range of 70°C to 150°C and a pressure of 0 atm to 5 atm, more preferably an external water stream is used to heat the process. This results in obtaining bioethanol with a maximum water content of 5%. Minimisation of water in the bioethanol stream minimises the reactor volume for fixed space velocity and bioethanol production.

In another preferred embodiment of the process, the solid acid catalyst is selected from γ-Al₂O₃, ZSM-5 type zeolites, silicoaluminophosphates (SAPO) and heteropolyacids (HPA), wherein said heteropolyacids are selected from those with a Keggin, Wells-Dawson, Preysler, Stromberg, and Anderson-Evans structure. In a more preferred embodiment, the solid acid catalyst is a heteropolyacid (HPA) of Keggin structure and is selected from phosphotungstic acid, silicotungstic acid, phosphomolybdic acid and silicomolybdic acid, preferably phosphotungstic acid, silicotungstic acid. In a still more preferred embodiment, the solid acid catalyst is supported on an oxide selected from SiO₂, mesoporous silicas, TiO₂, and ZrO₂. In an even more preferred embodiment, the optimal HPA load in the catalyst, understood as the relative amount of HPA that maximises ethylene production, is related to the specific area of the support with a ratio of 20 to 85% by weight.

In another preferred embodiment of the process, the solid acid catalyst may be in the form of powder, extruded, tablets and monoliths.

In another preferred embodiment of the process, the liquid/gas separation method of step (g) consists of cooling and condensing the liquid by-products, obtaining a stream of high purity bioethylene gas.

In another preferred embodiment of the process, the liquid/gas separation method of step (g) consists of a single distillation tower.

In another preferred embodiment of the process, the temperature of step (g) is between 205°C and 215°C, to maximise the service life of the catalyst.

In another preferred embodiment of the process, the pressure of step (g) is between 4 bar and 6 bar. This pressure is sufficient for optimal reaction operation and sufficient to obtain high purity bioethylene in the liquid/gas separation (g).

In another preferred embodiment of the process, the steps of the process are carried out by conventional continuous, semi-continuous, discontinuous techniques in a reactor selected from a fixed-bed reactor and fluidised bed reactor and the reactor operates in adiabatic or isothermal mode. In a more preferred embodiment, the reactor operates in isothermal mode because in this way the catalyst always operates at the optimum reaction temperature, whereas in a non-isothermal reactor, as the reaction is endothermic, it would result in a temperature profile that would decrease from the inlet to the outlet which forces the catalyst to operate outside the optimum operating zone.

Throughout the description and the claims, the word "comprises" and its variants do not intend to exclude other technical features, additives, components or steps. For those skilled in the art, other objects, advantages and features of the invention may be partially deduced from both the description and the embodiment of the invention. The following examples are provided by way of illustration and are not intended to limit the present invention.

### EXAMPLES

Next, the invention will be illustrated by means of assays carried out by the inventors that demonstrate the effectiveness of the product of the invention.

### Example 1

First, an organic fraction of municipal solid waste (OFMSW) from an MSW treatment plant comprising a 90% biodegradable fraction and a 10% fraction of inert materials (glass, plastics, textiles, stones, ferrous materials, etc.) by weight. The biodegradable fraction has a moisture content of 50% by weight and contents of glucan, xylan, protein and fat of 30%, 3%, 10% and 7% by weight, respectively, on a dry basis. This OFMSW is passed through a grinder which reduces its particle size to 2-4 cm and a conveyor belt where there is an in-line magnetic separator to remove ferrous materials. Treat with sulphuric acid by heating to a temperature of 130°C at a concentration of 1% by volume and a residence time of 60 minutes. The product obtained is mixed with water at a percentage of solids of 20% and subjected to wet grinding by a blade system to a particle size of less than 10 mm. This aqueous mixture is heated to 50°C and the pH is adjusted to between 4.5-5.5. A 0.5% by volume is added with respect to the acid mixture obtained from the enzyme cocktail of the supplier Novozymes with cellulase and amylase activities for saccharification for 24 hours. Afterwards, the temperature is reduced to 30-38°C, 1 g/l of yeast is added and simultaneous saccharification and fermentation (36h) is carried out, resulting in the fermentation of the sugars generated to bioethanol, at a concentration of 3% by weight. During the process, non-biodegradable materials contained in the mixture are separated by means of a float retention system with a perforated sieve of 8-10 mm, which separates lightweight materials (plastics, textiles, corks, fibres) combined with a filter at the bottom of the 8-12 mm mesh light tank which retains the heavier materials (glass, stones, sand, metals). The floating materials are removed automatically while the heavy materials are removed after decanting and emptying of the tank by means of an automatic system. The fermented broth after simultaneous saccharification and fermentation containing the diluted bioethanol is subjected to solid/liquid separation (S/L) by mechanical pressure methods (rotating trommel + screw press) where a liquid stream (10% solids) and a solid stream (52% solids) are obtained. The liquid stream is sent to a distillation unit to concentrate the bioethanol generated. The liquid stream resulting from the foregoing S/L separation step is subjected to a distillation process until an ethanol of more than 95% m/m purity is obtained, which will be used as a chemical brick for the production of bioethylene. The advanced bioethanol to ethylene dehydration process is characterised by putting the bioethanol in gas phase in contact with a solid acid catalyst, as a phosphotungstic acid supported on SiO₂. The dehydration reaction results in a gas stream of mostly bioethylene. The temperature of the reaction is 220°C and the pressure at which this reaction takes place is 5 bar. The catalyst activity for the ethanol dehydration reaction is very high and has a high selectivity towards ethylene production; it preferably works with an ethanol conversion above 90% and a selectivity towards ethylene of more than 90%. The reaction products do not include CO or CO₂. Conversion and production levels above 90% are achieved. Next, the dehydration compounds are separated. This separation is carried out by means of separation techniques known in the state of the art, specifically, liquid-gas separation. The absence of carbon oxides among the reaction products simplifies the required separation processes.

## Claims

1. A process for obtaining bioethylene from an organic fraction of municipal solid waste, **characterised in that** it comprises the following steps:
a) pretreating the organic fraction mechanically of municipal solid waste to reduce its particle size to between 1 and 6 cm, preferably between 1 cm and 4 cm;
b) mixing with an inorganic mineral acid in a concentration of between 0.5 and 3% by volume of the organic fraction of municipal solid waste obtained in step (a) and heating for 30 to 90 minutes at a temperature of between 100°C and 200°C;
c) mixing the product obtained in (b) with water in a percentage of solids of between 8 and 35% and subsequently grinding to a particle size of less than 10 mm, heating between 40°C and 70°C and adjusting the pH between 4 and 6
d) adding an enzyme cocktail comprising at least one cellulase and one amylase to the acid solution obtained in (c) between 0.1% and 2% by weight of said acid solution obtained in (c), and let it react for 3 to 24 hours, subsequently reducing the temperature to between 30 and 40°C, adding yeasts (0.1-1 g/l) and carrying out simultaneous fermentation and saccharification for 24 to 48 hours, wherein floating materials and heavy materials are additionally separated during fermentation and saccharification;
e) separating the broth obtained in (d) by means of a solid/liquid method;
f) reducing the water content of the liquid obtained in (e) by distillation; and
g) putting the bioethanol obtained in (f) in gaseous form in the absence of any additional eluent or carrier gas in contact with a solid acid catalyst at a temperature of between 190°C and 250°C and a pressure of between 1 and 10 bar, and separating the dehydration compounds obtained by means of the liquid/gas separation method.

2. The process according to claim 1, wherein the inorganic acid of step (b) is selected from sulphuric acid, nitric acid, hydrochloric acid, phosphoric acid and any combination thereof.

3. The process according to any of claims 1 or 2, wherein the solid/liquid separation of step (e) is carried out by a mechanical pressure method based on a rotating trommel screen and screw press.

4. The process according to any of claims 1 to 3, wherein the distillation of step (f) is carried out by a 2- to 4-column system in a temperature range of 70°C to 150°C and a pressure of 0 atm to 5 atm.

5. The process according to any of claims 1 to 4, wherein the solid acid catalyst is selected from γ-Al₂O₃, ZSM-5 type zeolites, silicoaluminophosphates (SAPO) and heteropolyacids (HPA), wherein said heteropolyacids are selected from those with a Keggin, Wells-Dawson, Preysler, Stromberg, and Anderson-Evans structure.

6. The process according to claim 5, wherein the solid acid catalyst is a heteropolyacid (HPA) of Keggin structure and is selected from phosphotungstic acid, silicotungstic acid, phosphomolybdic acid and silicomolybdic acid.

7. The process according to claim 6, wherein the solid acid catalyst is supported on an oxide selected from SiO₂, mesoporous silicas, TiO₂, and ZrO₂.

8. The process according to claim 7, wherein the optimal HPA load in the catalyst, understood as the relative amount of HPA that maximises ethylene production, is related to the specific area of the support with a ratio of 20 to 85% by weight.

9. The process according to any of claims 1 to 8, wherein the solid acid catalyst may be in the form of powder, extruded, tablets and monoliths.

10. The process according to any of claims 1 to 9, wherein the liquid/gas separation method consists of cooling and condensing the product gas obtained in (g).

11. The process according to any of claims 1 to 10, wherein the temperature of step (g) is between 205°C and 215°C.

12. The process according to any of claims 1 to 11, wherein the pressure of step (g) is between 4 bar and 6 bar.

13. The process according to any of claims 1 to 12, wherein the steps of the process are carried out by conventional continuous, semi-continuous, discontinuous techniques in a reactor selected from a fixed-bed reactor and fluidised bed reactor and the reactor operates in adiabatic or isothermal mode.

14. The process according to claim 13, wherein the reactor operates in isothermal mode.
